# FASCICULE DE BREVET EUROPEEN

(11) **EP 0 515 541 B1**
(45) Date de publication et mention de la délivrance du brevet: **27.04.1994**
(21) Numéro de dépôt: 91905073.2
(22) Date de dépôt: 20.02.1991
(51) Int. Cl.: C07D 303/48, C07B 57/00, C07D 305/14

(54) **PROCEDE DE PREPARATION DE L'ACIDE CIS-$g(b)-PHENYLGLYCIDIQUE-(2R,3R)**
VERFAHREN ZUR HERSTELLUNG VON (2R,3R)-CIS-BETA-PHENYL-GLYCIDIL-SÄURE
METHOD FOR PREPARING CIS-$g(b)-PHENYLGLYCIDIC-(2R,3R) ACID

(30) Priorité: 21.02.1990 FR 9002098
(43) Date de publication de la demande: 02.12.1992
(73) Titulaire: RHONE-POULENC RORER S.A., 92165 Antony Cédex (FR)
(72) Inventeur: DUCHESNE, Jean-Pierre, F-69007 Lyon (FR); MULHAUSER, Michel, F-69130 Ecully (FR)
(74) Mandataire: Pilard, Jacques
(86) Numéro de dépôt international: FR9100133
(87) Numéro de publication internationale: WO9113066

(56) Documents cités:
- EP-A- 0 253 738
- EP-A- 0 253 739
- EP-A- 0 336 840
- EP-A- 0 336 841
- EP-A- 0 342 903
- EP-A- 0 414 610
- Bulletin of the Chemical Society of Japan, Vol. 47, No. 11, November 1974, (Tokyo, K. Harada et al.: "Optical resolution and configuration of cis-beta-phenyl-glycidic acid", pages 2911, 2912, see the whole article cited in the application
- Journal of Organic Chemistry, Vol. 31, No. 5, May 1966, (Easton, US), K. Harada:"Optical resolution and absolute configuration of trans-beta-phenylglycidic acid", pages 1407-1410, see the whole article cited in the application
- Journal of Organic Chemistry, Vol. 51, No. 1, January 1986, American Chemical Society, (Easton, US), J.-N. Denis et al.: ""An efficient, enantioselective synthesis of the taxolside chain", pages 46-50, see the whole article; in particular page 48, the paragraph spanning the two columns, cited in the application
- Journal of the American Chemical Society, Vol. 110, No. 17, 17 August 1988, American Chemical Society, (Washington, DC, US), J.-N. Denis et al.: "A highly efficient practial approach to natural taxol", page 5917-5919, see the whole article cited in the application

## Description

La présente invention concerne un nouveau procédé de préparation de l'acide β-phénylglycidique-(2R,3R) de formule : 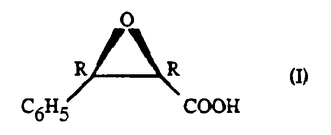
éventuellement sous forme de sel ou d'ester.

Les produits de formule générale (I) peuvent être utilisés pour préparer le taxol dans les conditions décrites dans les articles de J-N. Denis et coll., J. Org. Chem., 51, 46-50 (1986) ; J. Amer. Chem. Soc., 110, 5917-5919 (1988) ou les dérivés du taxol qui sont décrits dans le brevet européen EP 253 738.

Il est connu, en particulier d'après J-N. Denis et coll., J. Org. Chem., 51, 46-50 (1986) de préparer les produits de formule générale (I) par époxydation asymétrique catalysée au titane [T. Katsuki et K.B. Sharpless, J. Amer. Chem. Soc., 102, 5974-5976 (1980) ; J.G. Hill et coll., J. Org. Chem., 48, 3607 (1983)] de l'alcool cinnamique cis suivie de l'oxydation et de l'estérification de l'époxyalcool obtenu. Cependant, les rendements ne sont pas satisfaisants, les excès énantiomériques sont généralement inférieurs à 80 % et la voie est relativement longue.

Selon K. Harada, J. Org. Chem., 31, 1407-1410 (1966), il est connu de séparer les isomères de l'acide trans-β-phénylglycidique par précipitation d'un sel avec l'α-méthylbenzylamine optiquement active.

Selon K. Harada et Y. Nakajima, Bull. Chem. Soc. Japan, 47 (11) 2911-2912 (1974) il est connu de séparer les isomères de l'acide cis-β-phénylglycidique par précipitation d'un sel avec l'éphédrine optiquement active.

Dans la demande EP 0 342 903 est décrite la séparation d'un énantiomère de l'acide β-(méthoxy-4 phényl) glycidique à partir du diastéréoisomère correspondant au moyen de l'α-méthylbenzylamine optiquement active.

Cependant, pour que ces procédés puissent être mis en oeuvre, il est nécessaire de séparer préalablement les isomères cis et trans de l'acide β-phénylglycidique.

Il a maintenant été trouvé, et c'est ce qui fait l'objet de la présente invention, que l'acide β-phénylglycidique-(2R,3R) peut être obtenu à partir d'un mélange des isomères cis et trans.

Le procédé selon l'invention consiste à cristalliser dans un solvant convenable le sel de l'acide β-phénylglycidique-(2R,3R) avec la (+)-α-méthylbenzylamine-(R) contenu dans un mélange des sels de la (+)-α-méthylbenzylamine-(R) avec l'acide β-phénylglycidique- (2R,3R), l'acide β-phénylglycidique-(2S,3S), l'acide β-phénylglycidique-(2R,3S) et l'acide β-phénylglycidique-(2S,3R).

Comme solvants peuvent être utilisés l'eau ou un solvant organique choisi parmi les alcools aliphatiques contenant 1 à 4 atomes de carbone tels que le méthanol ou l'éthanol, les éthers ou les cétones éventuellement en mélange avec de l'eau.

Généralement, la précipitation du sel de l'acide β-phénylglycidique-(2R,3R) avec la (+)-α-méthylbenzylamine-(R) est effectuée par addition d'acétone à une solution aqueuse ou organique, de préférence éthanolique, du mélange des sels des isomères optiques des acides β-phénylglycidiques cis et trans avec la (+)-α-méthylbenzylamine.

Lorsque l'on opère en milieu organique, il est particulièrement avantageux d'ajouter l'acétone à la solution organique au reflux puis de faire précipiter le sel désiré par refroidissement.

Lorsque l'on opère en milieu aqueux, il est particulièrement avantageux d'ajouter l'acétone jusqu'à l'obtention d'un mélange contenant de 25 à 50 % d'eau et de 75 à 50 % d'acétone. De préférence, les meilleurs résultats sont obtenus lorsque la solution de cristallisation contient environ 35 % d'eau et 65 % d'acétone.

Le mélange des sels des isomères optiques des acides β-phénylglycidiques cis et trans avec la (+)-α-méthylbenzylamine-(R) peut être obtenu:
- par action de la (+)-α-méthylbenzylamine-(R) sur le mélange des isomères cis et trans de l'acide β-phénylglycidique préparé in situ, ou bien
- par action d'un sel de la (+)-α-méthylbenzylamine-(R), tel que le chlorhydrate, sur le mélange des sels alcalins, tel que les sels de potassium, des acides β-phénylglycidiques cis et trans.

Le mélange des sels alcalins des acides β-phénylglycidiques cis et trans peut être obtenu par saponification des esters correspondants au moyen d'une base minérale. Il est particulièrement avantageux d'utiliser la potasse éthanolique en opérant à une température voisine de 20 C. Il n'est pas nécessaire d'isoler les esters préalablement à l'action de la base minérale.

Le mélange des acides β-phénylglycidiques cis et trans peut être obtenu in situ par acidification d'une solution aqueuse du mélange des sels alcalins correspondants.

Le mélange des esters des acides β-phénylglycidiques cis et trans peut être obtenu par action d'un halogénoacétate d'alkyle, tel qu'un chloro- ou un bromoacétate d'alkyle, sur le benzaldéhyde selon la méthode décrite par F.W. Bacheler et R.K. Bansal, J. Org. Chem., 34 (11) 3600-3604 (1969). Il est particulièrement avantageux d'utiliser le chloroacétate de t.butyle qui permet d'obtenir un mélange pratiquement équimoléculaire des isomères cis et trans.

En remplaçant le chloroacétate de t.butyle par le bromoacétate de t.butyle et en opérant, de préférence, au voisinage de 0 C, il est possible d'obtenir un mélange dans lequel la proportion d'isomère cis est voisine de 75 %.

Par action d'une base minérale, de préférence la potasse éthanolique, sur l'ester préparé in situ comme indiqué ci-dessus, il est possible de précipiter le mélange des sels alcalins des acides β-phénylglycidiques cis et trans (sels de potassium) en association avec l'halogénure alcalin (chlorure ou bromure de potassium) qui peut être traité, après isolement, par une solution aqueuse d'un sel de la (+)-α-méthylbenzylamine-(R) comme indiqué précédemment.

Quel que soit le procédé mis en oeuvre pour préparer le mélange des esters des acides cis et trans, il est possible d'obtenir un mélange contenant 80 % d'ester cis et 20 % d'ester trans par cristallisation de l'isomère trans dans l'isomère cis qui joue le rôle de solvant.

L'acide β-phénylglycidique-(2R,3R) obtenu selon le procédé de la présente invention est particulièrement utile pour préparer les dérivés du taxane de formule générale : 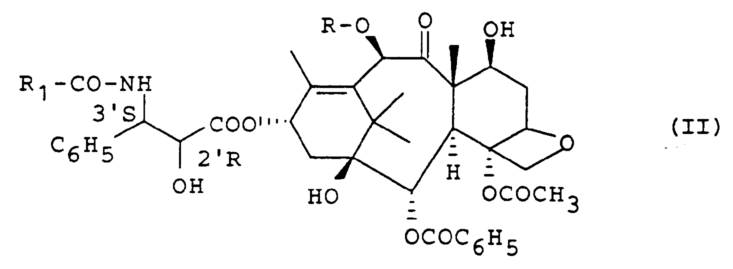
dans laquelle R représente un atome d'hydrogène ou un radical acétyle et R₁ représente un radical phényle ou t.butoxy.

Les exemples suivants, donnés à titre non limitatif, montrent comment l'invention peut être mise en pratique.

### EXEMPLE 1

a) Dans un ballon de 2 litres, on introduit 106 g de benzaldéhyde (1 mole), 150,7 g de chloroacétate de t.butyle (1 mole) et 450 cm3 de t.butanol. On ajoute ensuite en 2 heures 40 minutes, à une température comprise entre 18 et 24 C, une solution de 112,5 g de t.butylate de potassium (1 mole) dans 850 cm3 de t.butanol. Après 18 heures d'agitation à une température voisine de 20 C, le t.butanol est évaporé sous pression réduite. Le résidu obtenu est repris par 1000cm3 d'eau. On extrait par 3 fois 300 cm3 de chlorure de méthylène. Les phases organiques réunies sont séchées sur sulfate de magnésium. Après filtration et concentration à sec sous pression réduite, on obtient une huile (210 g) dont la composition molaire, déterminée par le spectre de résonance magnétique nucléaire du proton, est la suivante :
   cis-β-phénylglycidate de t.butyle : 53 %
   trans-β-phénylglycidate de t.butyle : 42 %
   chloroacétate de t.butyle : 5 %
b) En opérant comme précédemment mais en remplaçant le chloroacétate de t.butyle par 195 g de bromoacétate de t.butyle (1 mole) on obtient une huile (213 g) dont la composition molaire, déterminée par le spectre de résonance magnétique nucléaire du proton, est la suivante :
   cis-β-phénylglycidate de t.butyle : 63 %
   trans-β-phénylglycidate de t.butyle : 32 %
   bromoacétate de t.butyle : 5 %

Dans un réacteur, on introduit 800 cm3 d'éthanol et 298 g d'un mélange de cis-β-phénylglycidate de t.butyle (80 %) et de trans-β-phénylglycidate de t.butyle (20 %) obtenu après cristallisation à 4 C pendant plusieurs jours et séparation de l'isomère trans dans le mélange des isomères cis et trans obtenu précédemment en a) ou b).

La solution éthanolique est refroidie à une température voisine de 0 C puis est traitée en 3 heures par 134 g de potasse à 85% (2,03 mole) en solution dans 600 cm3 d'éthanol. Après 18 heures d'agitation, le mélange réactionnel est filtré. Le solide obtenu est lavé par 200 cm3 d'éthanol froid puis séché à poids constant. On obtient ainsi 185 g d'un solide blanc dont la composition, déterminée par le spectre de résonance magnétique nucléaire du proton, est la suivante :
cis-β-phénylglycidate de potassium : 74 %
trans-β-phénylglycidate de potassium : 42 %

Dans un réacteur, on introduit :
- 185 g de β-phénylglycidate de potassium obtenu précédemment
- 625 cm3 d'un mélange glace-eau
- 770 cm3 d'éther éthylique

Le mélange est refroidi à 0 C. On ajoute, à cette température, en 2 heures, 185 cm3 d'acide chlorhydrique 5N. Dès la fin de l'addition, les phases organique et aqueuse sont séparées par décantation. La phase aqueuse est extraite avec 2 fois 100 cm3 d'éther. Les phases organiques sont séchées sur sulfate de sodium. Après filtration, les phases organiques sont traitées, sous forte agitation, par 125 cm3 de (+)-α-méthylbenzylamine-(R) (0,95 mole). Le précipité formé est séparé par filtration, lavé par 200 cm3 d'éther éthylique froid puis séché. On obtient ainsi 194 g d'une poudre blanche, dont l'analyse par le spectre de résonance magnétique nucléaire du proton, montre qu'elle est constituée de 81 % de cis-β-phénylglycidate de (+)-α-méthylbenzylamine-(R) et de 19 % de trans-β-phénylglycidate de (+)-α-méthylbenzylamine-(R).

Dans un réacteur, on introduit 193 g du sel obtenu précédemment et 800 cm3 d'éthanol. Le mélange est chauffé au reflux. On obtient ainsi une solution homogène incolore qui est traitée au reflux par 1600 cm3 d'acétone. On laisse refroidir la solution homogène jusqu'à 45 C puis on ajoute quelques cristaux de β-phénylglycidate-(2R,3R) de (+)-α-méthylbenzylamine-(R). Dès que la température atteint 42 C, il se forme un précipité abondant. Deux heures après la fin de l'addition de l'acétone, la température est voisine de 25 C. Le précipité est séparé par filtration, rincé à l'acétone et séché à poids constant. On obtient ainsi 36 g de β-phénylglycidate-(2R,3R) de (+)-α-méthylbenzylamine-(R) (0,126 mole) pratiquement pur.

L'excès énantiomérique mesuré après dérivation en ester méthylique suivie d'une analyse par CLHP chirale est de 97 %.

### EXEMPLE 2

Dans un réacteur, on introduit 28,53 g de β-phénylglycidate-(2R,3R) de (+)-α-méthylbenzylamine-(R) (0,1 mole) obtenu à l'exemple 1, et 200 cm3 de dichlorométhane. Au mélange hétérogène, on ajoute en 20 minutes, à 22 C, 50 cm3 de potasse 2N. Dès la fin de l'addition, les deux phases liquides sont séparées par décantation. La phase organique est lavée à l'eau. Les phases aqueuses réunies sont concentrées à sec. On obtient ainsi 19,9 g de β-phénylglycidate de potassium-(2R,3R) dont le pouvoir rotatoire est [α]_{D} = -2,8 (c = 7,4; eau).

### EXEMPLE 3

Dans un ballon de 6 litres, on introduit 523 cm3 de (+)-α-méthylbenzylamine-(R) (3,97 moles) et 760 cm3 d'éthanol. Le mélange réactionnel est refroidi extérieurement par un mélange glace-acétone. On ajoute, en maintenant la température inférieure à 10 C, 143 cm3 d'acide chlorhydrique 2,78N. Le mélange réactionnel reste limpide et incolore.

Dans un ballon de 10 litres, on introduit 3,8 litres d'éthanol et 768 g d'un produit dont la composition est la suivante :
cis-β-phénylglycidate de potassium : 80 %
trans-β-phénylglycidate de potassium : 12 %
chloroacétate de potassium : 8 %

Au mélange hétérogène ainsi obtenu, chauffé à 35-40 C, on ajoute la solution de chlorhydrate de (+)-α-méthylbenzylamine-(R) préparée ci-dessus. Le mélange réactionnel est maintenu à 50 C pendant 2 heures. Le chlorure de potassium qui précipite est séparé par filtration et est lavé par du méthanol bouillant. Le filtrat est concentré jusqu'à un poids de 2445 g puis laissé pendant 18 heures à température ambiante. Les cristaux apparus sont séparés par filtration, lavés 6 fois par 200 cm3 d'éthanol et séchés à poids constant. On obtient ainsi 530 g de cristaux dont l'analyse par le spectre de résonance magnétique nucléaire du proton montre qu'il est constitué de cis-β-phénylglycidate de (+)-α-méthylbenzylamine-(R) pur dont l'excès énantiomérique est de 7 %.

On place 525 g du sel ainsi obtenu dans un réacteur contenant 3,15 litres d'éthanol. Le mélange est chauffé au reflux de façon à devenir pratiquement homogène. On ajoute alors 6,3 litres d'acétone bouillant et quelques cristaux de sel (2R,3R). On laisse refroidir lentement à température ambiante. Les cristaux apparus sont séparés par filtration, lavés avec 6 fois 200 cm3 d'acétone et séchés à poids constant.

On obtient ainsi 217 g de β-phénylglycidate-(2R,3R) de (+)-α-méthylbenzylamine-(R) dont l'excès énantiomérique est de 98,4 %.

### EXEMPLE 4

a) Dans un réacteur de 170 litres en acier inoxydable, on dissout, sous atmosphère d'azote, à 30 C, 17,6 kg de t.butylate de potassium dans un mélange de 60 litres de t.butanol et de 65 litres de tétrahydrofuranne. On obtient ainsi 134 litres d'une solution A.
   Dans un réacteur de 250 litres, on introduit sous atmosphère d'azote, à 20 C, 28,720 kg de bromoacétate de t.butyle et 16,2 kg de benzaldéhyde dans 125 litres de t.butanol. On refroidit à 0 C puis on ajoute en 3 heures la solution A en maintenant la température à 0 C. On maintient à 0 C pendant encore 2 à 3 heures.
   A la solution obtenue, maintenue à 0 C, on ajoute, en 1 heure 20 minutes, une solution de 12,480 kg de potasse en pastilles dans 50 litres d'éthanol absolu. On maintient pendant environ 20 heures à 20 C tout en agitant fortement (150 tours/minute). On ajoute alors 24 litres d'eau dismutée puis chauffe à 50 C en 1 heure 30 minutes puis maintient pendant 10 minutes à cette température. On refroidit à -10 C en 6 heures puis filtre sous une pression d'azote de 2 bars. Le gâteau de filtration est lavé par 3 fois 30 litres puis par 20 litres d'un mélange méthyltertiobutyléther-éthanol (1-1 en volumes) puis séché sous pression réduite (1 mm de mercure ; 0,13 kPa) à 30 C. On obtient ainsi 45,58 kg d'un produit contenant, d'après le spectre de résonance magnétique nucléaire du proton, 57 % en poids d'un mélange des sels de potassium des acides β-phénylglycidique cis (73 %) et trans (27 %) et 43 % en poids de bromure de potassium.
b) Dans un réacteur de 100 litres, on introduit, sous atmosphère d'azote, 31,86 kg de (+)-α-méthylbenzylamine-(R) et 12 kg de glace. On refroidit extérieurement à -20 C puis on ajoute en 2 heures 23,64 litres d'acide chlorhydrique concentré (10,9N) en maintenant la température du mélange réactionnel comprise entre 20 et 25 C. On obtient ainsi 65 litres d'une solution limpide incolore.

Dans un réacteur de 250 litres, on introduit, sous atmosphère d'azote, 87,3 kg du mélange des sels de potassium des acides β-phénylglycidiques cis et trans et du bromure de potassium obtenu dans les conditions décrites précédemment et 200 litres d'eau distillée. On chauffe à 45 C jusqu'à dissolution puis refroidit à 30 C. On ajoute, en 10 minutes, 20 litres de solution de chlorhydrate de (+)-α-méthylbenzylamine-(R). On amorce la cristallisation avec 13 g de sel cis-(R,R). La cristallisation est instantanée. On ajoute alors, en 15 minutes, le reste de la solution de chlorhydrate de (+)-α-méthylbenzylamine-(R).

Après 1 heure de refroidissement à 22 C, on ajoute 17,5 kg de chlorure de sodium et agite pendant 3 heures. La température est de 17°C. On filtre sous une pression d'azote de 2 bars. On obtient 263 litres de filtrat et 60 litres de gâteau. Le gâteau est lavé avec 2 fois 40 litres d'une solution aqueuse de chlorure de sodium à 340g/litre puis 25 litres de solution de chlorure de sodium. On obtient 118 litres de lavages et 48 litres de gâteau. Le gâteau est battu sur filtre avec 50 litres d'eau à 0 C pendant 1 heure. On obtient 55 litres de filtrat et 45 litres de gâteau qui est séché à poids constant. On obtient ainsi 34,1 kg de sel de l'acide cis-β-phénylglycidique avec la (+)-α-méthylbenzylamine-(R) pratiquement pur (chromatographie liquide en phase vapeur avec dérivatisation, spectre de résonance magnétique nucléaire du proton à 200 MHz, dosage potentiométrique).

Dans un réacteur émaillé de 250 litres, on introduit 51,2kg d'eau et 36,4 litres d'acétone puis 33,7 kg de sel de l'acide cis-β-phénylglycidique avec la (+)-α-méthylbenzylamine-(R), titrant 95% et contenant 53,2 % de produit 2R,3R, soit 60,4 moles.

Le mélange réactionnel est chauffé au reflux (63°C) puis refroidi lentement après amorçage par addition de 58 g de sel 2R,3R pur.

Après quelques heures à une température voisine de 20 C, les cristaux sont séparés par filtration, lavé par 3 fois 15 litres d'un mélange eau-acétone (36-64 en volumes) puis par 3 fois 15 litres d'acétone. Après séchage à poids constant sous pression réduite (5mbar) à 30 C, on obtient 12,5 kg de sel de l'acide cis-β-phénylglycidique-(2R,3R) avec l'α-méthylbenzylamine-(R) pur d'après l'analyse par CHLP chirale.

Le rendement est de 72 %.

## Revendications

1. Procédé de préparation de l'acide cis-b-phénylglycidique-(2R,3R) de formule : 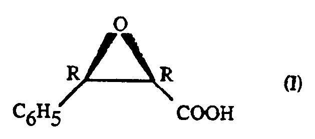 éventuellement sous forme de sel ou d'ester, caractérisé en ce que l'on cristallise sélectivement dans un solvant convenable le sel de l'acide β-phénylglycidique-(2R,3R) avec la (+)-α-méthylbalzylamine-(R) dans une solution d'un mélange des sels de la (+)-α-méthylbenzylamine-(R) avec les acides β-phénylglycidique-(2R,3R), β-phénylglycidique-(2S,3S), β-phénylglycidique-(2R,3S) et β-phénylglycidique-(2S,3R), et isole le produit obtenu et le transforme éventuellement en sel de métal alcalin ou en ester.

2. Procédé selon la revendication 1 caractérisé en ce que le solvant est choisi parmi l'eau et les solvants organiques choisis parmi les alcools aliphatiques contenant 1 à 4 atomes de carbone, les éthers et les cétones éventuellement en mélange avec l'eau.

3. Procédé selon l'une des revendications 1 ou 2 caractérisé en ce que l'on ajoute de l'acétone à une solution aqueuse, organique ou hydroorganique du mélange des sels définis dans la revendication 1, puis précipite le sel de l'acide β-phénylglycidique-(2R,3R) avec la (+)-α-méthylbenzylamine-(R) et l'isole par filtration.

4. Procédé selon l'une des revendications 1 à 3 caractérisé en ce que l'on utilise un mélange constitué d'environ 80 % des sels de l'isomère cis de l'acide β-phénylglycidique avec la (+)-α-méthylbenzylamine-(R) et d'environ 20 % des sels de l'isomère trans de l'acide β-phénylglycidique avec la (+)-α-méthylbenzylamine-(R).

5. Procédé selon l'une des revendications 1 à 4 caractérisé en ce que le mélange des sels de la (+)-α-méthylbenzylamine-(R) avec les isomères cis et trans de l'acide β-phénylglycidique est obtenu par action de la (+)-α-méthylbenzylamine sur un mélange des isomères cis et trans de l'acide β-phénylglycidique préparé in situ.

6. Procédé selon l'une des revendications 1 à 4 caractérisé en ce que le mélange des sels de la (+)-α-méthylbenzylamine-(R) avec les isomères cis et trans de l'acide β-phénylglycidique est obtenu par action d'un sel de la (+)-α-méthylbenzylamine sur le mélange des sels alcalins des acides β-phénylglycidiques cis et trans éventuellement préparés in situ.

## Claims

1. Process for the preparation of (2R,3R)-cis-β-phenylglycidic acid of formula: 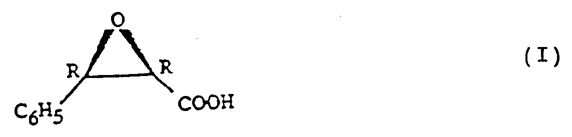 optionally in the salt or ester form, characterised in that the salt of (2R,3R)-β-phenylglycidic acid with (R)-(+)-α-methylbenzylamine in a solution of a mixture of the salts of (R)-(+)-α-methylbenzylamine with (2R,3R)-β-phenylglycidic, (2S,3S)-β-phenylglycidic, (2R,3S)-β-phenylglycidic and (2S,3R)-β-phenylglycidic acids is selectively crystallised in a suitable solvent, and the product obtained is isolated and optionally converted to an alkali metal salt or to an ester.

2. Process according to claim 1, characterised in that the solvent is chosen from water and organic solvents chosen from aliphatic alcohols containing 1 to 4 carbon atoms, ethers and ketones, optionally in a mixture with water.

3. Process according to one of claims 1 or 2, characterised in that acetone is added to an aqueous, organic or aqueous/organic solution of the mixture of the salts defined in claim 1, the salt of (2R,3R)-β-phenylglycidic acid with (R)-(+)-α-methylbenzylamine is then precipitated and isolated by filtration.

4. Process according to one of claims 1 to 3, characterised in that a mixture consisting of approximately 80 % of the salts of the cis isomer of β-phenylglycidic acid with (R)-(+)-methylbenzylamine and of approximately 20 % of the salts of the trans isomer of β-phenylglycidic acid with (R)-(+)-α-methylbenzylamine is used.

5. Process according to one of claims 1 to 4, characterised in that the mixture of the salts of (R)-(+)-α-methylbenzylamine with the cis and trans isomers of β-phenylglycidic acid is obtained by reacting (+)-α-methylbenzylamine with a mixture of the cis and trans isomers of β-phenylglycidic acid prepared in situ.

6. Process according to one of claims 1 to 4, characterised in that the mixture of the salts of (R)-(+)-α-methylbenzylamine with the cis and trans isomers of β-phenylglycidic acid is obtained by reacting a salt of (+)-α-methylbenzylamine with the mixture of the alkali metal salts of cis and trans β-phenylglycidic acids optionally prepared in situ.

## Patentansprüche

1. Verfahren zur Herstellung von (2R,3R)-cis-β-Phenylglycidsäure der Formel: 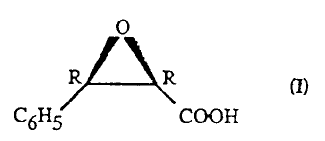 gegebenenfalls in Form des Salzes oder des Esters, dadurch gekennzeichnet, daß man selektiv in einem geeignetem Lösungsmittel das Salz der (2R,3R)-cis-β-Phenylglycidsäure mit (+)-(R)-α-Methylbenzylamin in einer Lösung eines Gemisches der Salze von (+)-(R)-α-Methylbenzylamin mit (2R,3R)-β-Phenylglycid-, (2S,3S)-β-Phenylglycid-, (2R,3S)-β-Phenylglycid-, und (2S,3R)-β-Phenylglycidsäure kristallisiert und das erhaltene Produkt isoliert und es gegebenenfalls in ein Alkalimetallsalz oder einen Ester überführt.

2. Verfahren gemäß Anspruch 1, dadurch gekennzeichnet, daß das Lösungsmittel unter Wasser und den organischen Lösungsmitteln, ausgewählt unter den aliphatischen Alkoholen mit 1 bis 4 Kohlenstoffatomen, den Ethern und den Ketonen, gegebenenfalls im Gemisch mit Wasser ausgewählt wird.

3. Verfahren gemäß einem der Ansprüche 1 oder 2, dadurch gekennzeichnet, daß man Aceton zu einer wässrigen organischen oder wässrig-organischen Lösung des Gemisches der in Anspruch 1 definierten Salze zugibt, hiernach das Salz der (2R,3R)-β-Phenylglycidsäure mit (+)-(R)-α-Methylbenzylamin ausfällt und es durch Filtration isoliert.

4. Verfahren gemäß einem der Ansprüche 1-3, dadurch gekennzeichnet, daß man ein Gemisch, bestehend aus etwa 80% der Salze des cis-Isomeren der β-Phenylglycidsäure mit (+)-(R)-α-Methylbenzylamin und etwa 20% der Salze des trans-Isomeren der β-Phenylglycidsäure mit (+)-(R)-α-Methylbenzylamin verwendet.

5. Verfahren gemäß einem der Ansprüche 1-4, dadurch gekennzeichnet, daß das Gemisch der Salze von (+)-(R)-α-Methylbenzylamin mit den cis- und trans-Isomeren der β-Phenylglycidsäure durch Umsetzung von (+)-α-Methylbenzylamin mit einem Gemisch der cis- und trans-Isomeren der β-Phenylglycidsäure, welches in situ hergestellt worden ist, erhalten wird.

6. Verfahren gemäß einemn der Ansprüche 1 bis 4, dadurch gekennzeichnet, daß das Gemisch der Salze von (+)-(R)-α-Methylbenzylamin mit den cis- und trans-Isomeren der β-Phenylglycidsäure durch Umsetzung eines Salzes von (+)-α-Methylbenzylamin mit dem Gemisch der Alkalisalze der cis- uns trans-β-Phenylglycidsäuren, welches gegebenenfalls in situ hergestellt worden ist, erhalten wird.
